# FASCICULE DE BREVET EUROPEEN

(11) **EP 0 456 545 B1**
(45) Date de publication et mention de la délivrance du brevet: **15.12.1993**
(21) Numéro de dépôt: 91401095.4
(22) Date de dépôt: 25.04.1991
(51) Int. Cl.: A61K 7/42, C07C 49/17, C07D 209/08

(54) **Association de dihydroxyacétone et de monohydroxyindoles pour conférer à la peau une coloration similaire au bronzage naturel, dispositif à plusieurs compartiments et procédé de mise en oeuvre**
Zusammensetzung von Dihydroxyaceton und Monohydroxyindolen, um der Haut eine der natürlichen Sonnenbräune ähnliche Farbe zu verleihen, Vorrichtung mit mehreren Fächern und Verfahren zu deren Anwendung
Composition of dihydroxyacetone and monohydroxyindoles to give the skin a natural sun tan colouration, device with several compartments and method of applicaton

(30) Priorité: 07.05.1990 FR 9005702
(43) Date de publication de la demande: 13.11.1991
(73) Titulaire: L'OREAL, 75008 Paris (FR)
(72) Inventeur: Cotteret, Jean, F-78480 Verneuil-sur-Seine (FR); Hourseau, Colette, F-93530 La Frette-sur-Seine (FR)
(74) Mandataire: Casalonga, Axel

(56) Documents cités:
- EP-A- 0 239 826
- DE-A- 3 930 446
- FR-A- 2 586 913
- GB-A- 2 184 017
- US-A- 3 920 808
- US-A- 4 515 773

## Description

L'invention est relative à l'association de dihydroxyacétone et de monohydroxyindoles pour conférer à la peau une coloration similaire à la coloration conférée à la peau par un bronzage naturel, ainsi qu'aux compositions et aux procédés mis en oeuvre.

La dihydroxyacétone ou DHA est connue depuis de nombreuses années dans son application à la coloration de la peau.

Il a également été proposé de colorer la peau en utilisant des composés indoliques et en particulier le 5,6-dihydroxyindole et certains de ses dérivés, ce qui fait l'objet de l'EP-A-239 826.

Ces différentes possibilités de conférer à la peau une coloration présentent cependant chacune des inconvénients.

La montée de la coloration par la DHA, due essentiellement à une réaction de type MAILLARD entre la DHA et les acides aminés de la peau, est lente. De plus, la coloration obtenue est généralement assez jaune et relativement éloignée de celle d'un bronzage naturel.

Par ailleurs, la coloration obtenue avec les dérivés indoliques précités, est également éloignée des nuances d'un bronzage naturel et présente de plus l'inconvénient de mal résister à l'eau.

Les inventeurs ont découvert, de façon surprenante, qu'en associant la dihydroxyacétone à certains monohydroxyindoles, il était possible d'obtenir une coloration intense se développant rapidement dans le temps, beaucoup plus proche, en nuance, de la coloration conférée à la peau par un bronzage naturel, que les colorations obtenues avec chacun des composés pris isolément. La coloration ainsi obtenue présente, par ailleurs, une très bonne ténacité dans le temps et en particulier au lavage.

Un objet de l'invention est donc constitué par l'association de dihydroxyacétone et d'au moins un monohydroxyindole sous forme libre ou salifiée, pour conférer à la peau une coloration similaire à celle résultant d'un bronzage naturel.

Un autre objet de l'invention est constitué par des compositions cosmétiques destinées à une application topique, contenant une telle association.

L'invention a également pour objet un procédé permettant de conférer à la peau une coloration similaire à celle résultant d'un bronzage naturel de la peau, par application de l'association précitée.

L'invention concerne enfin des dispositifs à plusieurs compartiments, contenant les différents composants de l'association définie ci-dessus.

D'autres objets de l'invention apparaîtront à la lecture de la description et des exemples qui suivent.

L'association, conforme à l'invention, destinée à conférer à la peau une coloration similaire à la coloration résultant du bronzage naturel, est essentiellement caractérisée par le fait qu'elle comprend :
un composant (A) contenant dans un milieu approprié pour une application topique, de la dihydroxyacétone, et
un composant (B) contenant dans un milieu appproprié pour la peau, au moins un monohydroxyindole, répondant à la formule : 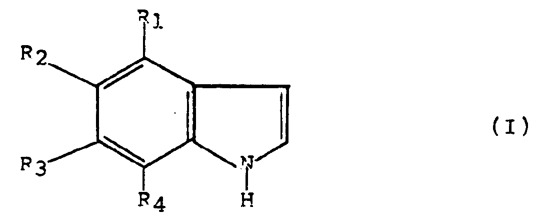
dans laquelle l'un des quatre substituants R₁, R₂, R₃ et R₄ désigne OH, les trois autres désignant hydrogène,
ainsi que les sels d'addition de ces monohydroxyindoles;
les composants (A) et (B) faisant partie d'une même composition ou étant destinés, soit à être mélangés tout juste avant l'emploi, soit à être appliqués de façon successive ou décalée dans le temps.

Comme sels d'addition de ces composés, on peut citer les chlorhydrates, sulfates et bromhydrates.

Une première variante de l'invention consiste à utiliser l'association conforme à l'invention, sous forme d'une composition unique, cette composition unique étant, soit préparée et stockée, soit mélangée tout juste avant l'emploi.

Dans ce cas, la dihydroxyacétone est utilisée dans des proportions comprises entre 0,1 et 10% en poids et en particulier entre 1 et 6%, par rapport au poids total de la composition contenant les composants (A) et (B), et le ou les monohydroxyindoles sont présents dans des proportions comprises entre 0.1 et 10%, et de préférence entre 0,1 et 5%, par rapport au poids total de la composition contenant les composants (A) et (B).

Selon une seconde variante, les composants (A) et (B) sont appliqués, soit de façon successive, soit de façon décalée dans le temps. Dans ce cas, la dihydroxy acétone est présente dans le composant (A) dans des proportions comprises de préférence entre 0.1 et 10% en poids par rapport au poids total de la composition (A) et en particulier entre 1 et 6%.

Le ou les monohydroxyindoles sont présents dans le composant (A) dans des proportions de préférence comprises entre 0.1 et et 10% en poids et en particulier entre 0,1 et 5% en poids par rapport au poids total du composant (B).

Le rapport en poids entre le monohydroxyindole de formule (I) et la dihydroxyacétone, est généralement supérieur à 0,01.

Le milieu cosmétiquement acceptable approprié pour une application topique, comprend généralement de l'eau, un mélange d'eau et d'un ou plusieurs solvant(s) organique(s), un mélange de solvants organiques ou encore des corps gras, cosmétiquement acceptables.

Ces compositions peuvent éventuellement être pressurisées dans les dispositifs aérosols en présence d'un agent propulseur, éventuellement en présence d'un générateur de mousse.

Les solvants utilisables sont choisis parmi les alcools inférieurs en C₁-C₄, tels que l'alcool éthylique, l'alcool isopropylique; les monoalcools saturés à longue chaîne ayant 10 à 18 atomes de carbone; les polyols ayant 2 à 8 atomes de carbone parmi lesquels on peut citer le glycérol; les alkylèneglycols comme l'éthylèneglycol, le propylèneglycol, le diéthylène glycol; les éthers de glycols parmi lesquels on peut citer les monoéthylèneglycol monoalkyléthers, les diéthylèneglycol monoalkyléthers, les triéthylène glycol monoalkyléthers, dans lesquels le groupement alkyle a de préférence 1 à 4 atomes de carbone, tels que par exemple l'éthylèneglycol monoéthyléther, l'éthylèneglycol monobutyléther, le diéthylèneglycol monoéthyléther; les esters acétiques de monoalkyl(C₁-C₃)éthers de l'éthylèneglycol, tels que l'acétate de monométhyléther de l'éthylèneglycol et l'acétate de monoéthyléther de l'éthylèneglycol; les esters d'acides gras saturés ayant de préférence 14 à 16 atomes de carbone et d'alcools saturés ayant de 1 à 4 atomes de carbone, parmi lesquels on peut citer le myristate d'isopropyle et le palmitate d'isopropyle.

Parmi les solvants énumérés ci-dessus, on préfère l'alcool éthylique, l'alcool cétylique, le propylèneglycol, l'éthylèneglycol monoéthyléther et l'éthylèneglycol monobutyléther.

Les solvants, lorsqu'ils sont utilisés dans le milieu aqueux, sont présents de préférence dans des proportions comprises entre 0,5 et 75% en poids et en particulier entre 2 et 50% en poids par rapport au poids total de la composition globale ou de chacun des composants (A) et (B).

Comme corps gras utilisables, on peut citer :
- les huiles et cires d'origine minérale, animale or végétale,
- les acides gras,
- les alcools gras, tels que laurique, cétylique, myristique, stéarique, palmitique, oléique,
- les esters d'acides gras, tels que le monostéarate de glycérol, le monostéarate de polyéthylèneglycol,
- les triglycérides d'acides gras en C₆-C₁₈.

Le milieu approprié pour une application topique peut être épaissi ou non. Pour l'épaissir, on peut utiliser les agents épaississants ou gélifiants bien connus dans l'état de la technique, tels que plus particulièrement la gomme de guar, les hétérobiopolysaccharides, tels que la gomme de xanthane et les scléroglucanes, les dérivés de cellulose, tels que la méthylcellulose, l'hydroxyéthylcellulose, l'hydroxypropylméthylcellulose, les sels de sodium de la carboxyméthylcellulose et les polymères d'acides acryliques préférentiellement réticulés.

Lorsque le milieu renferme de l'eau, le pH de la composition unique renfermant les composants (A) et (B) peut varier entre 3 et 10 et il est de préférence compris entre 3 et 7.

Lorsque les composants (A) et (B) sont utilisés séparément, le pH du composant (A) peut varier entre 3 et 10 et il est plus particulièrement compris entre 3 et 7, tandis que le pH du composant (B) peut varier entre 3 et 10 et il est compris plus particulièrement entre 4 et 7.

Les compositions constituées, soit par l'un et/ou l'autre des composants (A) et (B) ou par la composition contenant les deux composants (A) et (B), peuvent être utilisées sous forme de crèmes, de liquides plus ou moins épaissis, de dispersions vésiculaires de lipides amphiphiles ioniques ou non ioniques, d'huiles ou d'aérosols.

Les dispersions vésiculaires de lipides amphiphiles ioniques ou non ioniques peuvent être préparées selon des procédés connus.

On peut, par exemple, faire gonfler les lipides dans une solution aqueuse pour former des sphérules dispersées dans le milieu aqueux comme décrit dans l'article de BANGHAN, STANDISH, et WATKINS, J. mol. Biol., 13, 238 (1965) ou dans les brevets FR 2.315.991 et 2.416.008 de la demanderesse.

Ces compositions peuvent également contenir tous autres adjuvants utilisés habituellement dans les compositions cosmétiques pour la peau et plus particulièrement des agents tensio-actifs, de préférence non ioniques, des agents hydratants, adoucissants, des vitamines, des opacifiants, des parfums ou des agents conservateurs.

Les composants (A) et/ou (B) constituant la composition peuvent également contenir des filtres solaires filtrant le rayonnement UVB et/ou UVA.

A titre de filtres solaires, on peut citer les dérivés de l'acide cinnamique, le benzylidènecamphre et ses dérivés, l'acide paraaminobenzoïque et ses dérivés, les dérivés de l'acide salicylique, les dérivés de benzophénone, les homosalates, les dérivés du dibenzoylméthane.

Ces filtres sont présents dans des proportions allant de 0,2 à 10% en poids par rapport au poids du constituant (A) et/ou (B).

Les solvants, corps gras ou adjuvants utilisés dans les compositions de l'invention, ne doivent comporter ni groupements amine primaire, ni groupements oxydants.

Lorsque les composants (A) et (B) sont stockés séparément, l'association conforme à l'invention peut être conditionnée dans un dispositif à plusieurs compartiments encore appelé "kit" ou "nécessaire de coloration de la peau", comportant un premier compartiment contenant le composant (A) renfermant la dihydroxyacétone dans un milieu approprié pour une application topique et un second compartiment contenant le composant (B) renfermant, dans un milieu approprié pour une application topique, au moins un monohydroxy indole de formule (I) ou l'un de leurs sels, la dihydroxyacétone et le ou les monohydroxyindoles étant présents dans les proportions indiquées ci-dessus.

Une variante de ce dispositif peut consister à l'équiper d'un ensemble distributeur à double compartiment, tel que décrit par exemple par la demanderesse dans la demande de brevet français n° 2 586 913 et comportant deux poches séparées réunies dans un étui souple, les deux poches renfermant, pour l'une d'entre elles, le composant (A) tel que défini ci-dessus , et l'autre poche renfermant le composant (B) tel que défini ci-dessus.

Le procédé de traitement, en vue de conférer à la peau une coloration similaire à la coloration résultant d'un bronzage naturel de celle-ci, est essentiellement caractérisé par le fait que l'on applique le composant (A) et le composant (B) sur la peau, soit au moyen d'une seule composition éventuellement préparée tout juste avant l'emploi, soit en appliquant de façon successive le composant (A) et le composant (B) sur les parties de la peau destinées à être colorées, cette application pouvant éventuellement être décalée dans le temps de quelques minutes à plusieurs heures.

Conformément au procédé de l'invention, le composant (A) peut être appliqué avant ou après l'application du composant (B).

Les exemples suivants sont destinés à illustrer l'invention sans pour autant présenter un caractère limitatif.

### EXEMPLE 1

On prépare les compositions suivantes :

### COMPOSITION (A) :

- Dihydroxyacétone 5,0 g
- Alcool éthylique 15,0 g
- Gomme de xanthane 1,0 g
- Conservateur qs
- pH spontané 5,2
- Eau déminéralisée qsp 100,0 g

### COMPOSITION (B) :

- 5-hydroxyindole 0,5 g
- Alcool éthylique 15,0 g
- Gomme de xanthane 1,0 g
- Conservateur qs
- pH spontané 6,2
- Eau déminéralisée qsp 100,0 g

La coloration de la peau peut être obtenue selon les différents modes d'application suivants.

On applique la composition (A) puis la même quantité en poids de la composition (B) sur la peau.

On applique la composition (B) puis la même quantité en poids de la composition (A) sur la peau.

La coloration obtenue sur une peau non bronzée selon l'un quelconque de ces deux modes d'application est identique à celle d'un bronzage naturel.

### EXEMPLE 2

On prépare les compositions suivantes :

### COMPOSITION (A) :

- Dihydroxyacétone 5,0 g
- Alcool éthylique 15,0 g
- Gomme de xanthane 1,0 g
- Conservateur qs
- pH spontané 5,2
- Eau déminéralisée qsp 100,0 g

### COMPOSITION (B) :

- 6-hydroxyindole 0,5 g
- Alcool éthylique 15,0 g
- Gomme de xanthane 1,0 g
- Conservateur qs
- pH spontané 5,6
- Eau déminéralisée qsp 100,0 g

La coloration de la peau peut être obtenue selon les différents modes d'application suivants.

On applique la composition (A) puis la même quantité en poids de la composition (B) sur la peau.

On applique la composition (B) puis la même quantité en poids de la composition (A) sur la peau.

La coloration obtenue sur une peau non bronzée selon l'un quelconque de ces deux modes d'application est identique à celle d'un bronzage naturel.

### EXEMPLE 3

On prépare les compositions suivantes :

### COMPOSITION (A) :

- Dihydroxyacétone 5,0 g
- Alcool éthylique 15,0 g
- Gomme de xanthane 1,0 g
- Conservateur qs
- pH spontané 5,2
- Eau déminéralisée qsp 100,0 g

### COMPOSITION (B) :

- 4-hydroxyindole 1,0 g
- Alcool éthylique qsp 100,0 g

La coloration de la peau peut être obtenue selon les différents modes d'application suivants.

On applique la composition (A) puis la même quantité en poids de la composition (B) sur la peau.

On applique la composition (B) puis la même quantité en poids de la composition (A) sur la peau.

La coloration obtenue sur une peau non bronzée selon l'un quelconque de ces deux modes d'application est identique à celle d'un bronzage naturel.

### EXEMPLE 4

On prépare les compositions suivantes :

### COMPOSITION (A) :

- Dihydroxyacétone 5,0 g
- Alcool éthylique 15,0 g
- Gomme de xanthane 1,0 g
- Conservateur qs
- pH spontané 5,2
- Eau déminéralisée qsp 100,0 g

### COMPOSITION (B) :

- 7-hydroxyindole 1,0 g
- Alcool éthylique qsp 100,0 g

La coloration de la peau peut être obtenue selon les différents modes d'application suivantes.

On applique la composition (A) puis la même quantité en poids de la composition (B) sur la peau.

On applique la composition (B) puis la même quantité en poids de la composition (A) sur la peau.

La coloration obtenue sur une peau non bronzée selon l'un quelconque de ces deux modes d'application est identique à celle d'un bronzage naturel.

### EXEMPLE 5

### LOTION EPAISSIE

- Dihydroxyacétone 3,0 g
- 6-hydroxyindole 0,5 g
- Propylèneglycol 20,0 g
- Gomme de xanthane 0,25 g
- Conservateurs qs
- Eau purifiée qsp 100,0 g

### EXEMPLE 6

On prépare les compositions suivantes :

### COMPOSITION (A) :

Dans une première étape, on prépare un constituant (A1) comprenant les vésicules. On fond, en agitant doucement à une température de 95°C, un mélange de 3,8 g de lipides non ioniques, de formule : 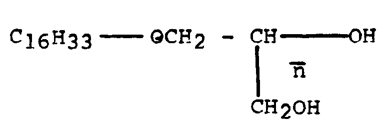
où n̅ est une valeur statistique moyenne égale à 3, avec 3,8 g de cholestérol, 0,4 g de sel monosodique du glutamate de stéaroyle vendu sous la dénomination ACYLGLUTAMATE HS 11 par la Société AJINOMOTO.

On introduit dans le mélange fondu 16 g d'eau portée à 90°C contenant un conservateur et l'on mélange pendant environ 5 minutes. A la phase ainsi obtenue, on ajoute 24 g d'eau à 20°C. On agite le mélange quelques minutes puis on complète par 2 g d'eau à 20°C et on affine le mélange par un passage dans un homogénéiseur haute pression à 500.10⁵ Pa (Rannie).

Dans une seconde étape, on prépare un constituant (A2) comprenant la phase aqueuse de dispersion :
- Dihydroxyacétone 2,0 g
- Paraméthoxycinnamate de 2-éthylhexyle vendu sous la dénomination ESCALOL 557 par la Société VAN DYK 0,5 g
- Glycérol 2,0 g
- Huile de vaseline 8,0 g
- Huile de silicone 5,0 g
- Hydroxyéthylcellulose vendu sous la dénomination NATROSOL PLUS GRADE 330 cs par la Société AQUALON 0,5 g
- Conservateurs, parfum qs
- Eau déminéralisée qsp 50,0 g

On réalise le mélange du constituant (A1) et du constituant (A2) et on homogénéise sous agitation douce.

### COMPOSITION (B) :

- 4-hydroxyindole 1,5 g
- Propylèneglycol 9,9 g
- Alcool éthylique 29,6 g
- Eau déminéralisée qsp 100,0 g

La coloration de la peau peut être obtenue selon le mode d'application suivant.

On applique un mélange équipondéral de la composition (A) et de la composition (B) sur la peau.

La coloration obtenue sur une peau non bronzée est identique à celle d'un bronzage naturel.

### EXEMPLE 7

On prépare les compositions suivantes :

### COMPOSITION (A) :

Le mode de préparation est identique à celui de l'exemple 1.

Lipides non ioniques de formule : 3,8 g 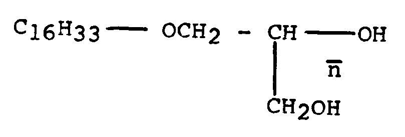
où n̅ est une valeur statistique moyenne égale à 3,
- Sel monosodique du glutamate de stéaroyle vendu sous la dénomination ACYLGLUTAMATE HS 11 par la Société AJINOMOTO 0,4 g
- Cholestérol 3,8 g
- Dihydroxyacétone 4,0 g
- Paraméthoxycinnamate de 2-éthylhexyle vendu sous la dénomination ESCALOL 557 par la Société VAN DYK 2,0 g
- 2-hydroxy 4-méthoxybenzophénone 0,5 g
- Glycérol 2,0 g
- Huile de vaseline 8,0 g
- Huile de silicone 5,0 g
- Hydroxyéthylcellulose vendu sous la dénomination NATROSOL PLUS GRADE 330 CS par la Société AQUALON 0,5 g
- Conservateurs, parfum qs
- pH spontané
- Eau déminéralisée qsp 100,0 g

### COMPOSITION (B) :

- 5-hydroxyindole 3,0 g
- Propylèneglycol 9,9 g
- Alcool éthylique 29,6 g
- Eau déminéralisée qsp 100,0 g

On applique un mélange équipondéral de la composition (A) et de la composition (B) sur la peau.

La coloration obtenue sur une peau non bronzée est identique à celle d'un bronzage naturel.

## Revendications

1. Association destinée à conférer à la peau une coloration similaire à la coloration résultant d'un bronzage naturel, caractérisée par le fait qu'elle comprend :
a) un composant (A) contenant dans un milieu approprié pour une application topique, de la dihydroxy acétone; et
b) un composant (B) contenant dans un milieu approprié pour une application topique, au moins un monohydroxyindole répondant à la formule : 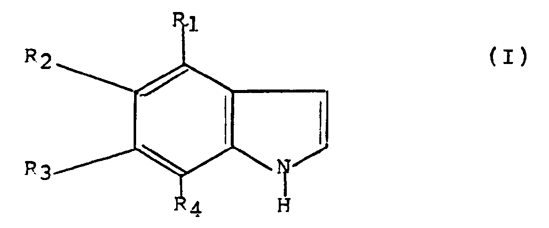 dans laquelle l'un des quatre substituants R₁, R₂, R₃ et R₄ désigne OH, les trois autres désignant hydrogène,
ainsi que les sels d'addition de ces monohydroxyindoles;
les composants (A) et (B) faisant partie d'une même composition ou étant destinés, soit à être mélangés tout juste avant l'emploi, soit à être appliqués de façon successive ou décalée dans le temps.

2. Association selon la revendication 1, caractérisée par le fait que les composants (A) et (B) constituent une seule composition contenant 0,1 à 10% en poids et de préférence 1 à 6% en poids de dihydroxyacétone et 0,1 à 10% en poids, de préférence 0,1 à 5% en poids de monohydroxyindole de formule (I), ces pourcentages étant exprimés par rapport au poids total de la composition.

3. Association selon la revendication 1, caractérisée par le fait que les composants (A) et (B) sont séparés, le composant (A) contenant la dihydroxy acétone dans des proportions comprises entre 0.1 et 10% en poids et en particulier entre 1 et 6% en poids par rapport au poids total du composant (A), le composant (B) contenant le monohydroxyindole de formule (I) dans des proportions comprises entre 0.1 et 10% en poids et de préférence entre 0,1 et 5% en poids par rapport au poids total de la composition (B).

4. Association selon l'une quelconque des revendications 1 à 3, caractérisée par le fait que le rapport en poids entre le monohydroxyindole de formule (I) et la dihydroxyacétone est supérieur à 0,01.

5. Association selon l'une quelconque des revendications 1 à 4, caractérisée par le fait que le milieu approprié pour l'application topique est un milieu comprenant de l'eau, un mélange d'eau et d'un ou plusieurs solvant(s) organique(s), un mélange de solvants organiques ou des corps gras, ces derniers et les solvants organiques étant cosmétiquement acceptables.

6. Application selon l'une quelconque des revendications 1 à 5, caractérisée par le fait que les solvants sont choisis parmi les alcools inférieurs en C₁-C₄, les monoalcools saturés à longue chaîne ayant 10 à 18 atomes de carbone, les polyols ayant 2 à 8 atomes de carbone, les alkylèneglycols, les éthers de glycols, les esters acétiques de monoalkyl(C₁-C₃)éthers de l'éthylèneglycol, les esters d'acides gras saturés ayant 14 à 16 atomes de carbone et d'alcools saturés ayant 1 à 4 atomes de carbone.

7. Association selon l'une quelconque des revendications 1 à 6, caractérisée par le fait que les solvants sont choisis parmi l'alcool éthylique, l'alcool cétylique, le propylèneglycol, l'éthylèneglycol mono éthyléther et l'éthylèneglycol monobutyléther.

8. Association selon l'une quelconque des revendications 1 à 7, caractérisée par le fait que les corps gras sont choisis parmi les huiles minérales, végétales ou animales, les acides gras, les alcools gras, les esters d'acides gras, les triglycérides d'acides gras.

9. Association selon l'une quelconque des revendications 1 à 8, caractérisée par le fait que les milieux appropriés pour une application topique sont épaissis.

10. Association selon l'une quelconque des revendications 1 à 9, caractérisée par le fait que le pH du composant (A) est compris entre 3 et 10 et de préférence entre 3 et 7 et que le pH du composant (B) est compris entre 3 et 10 et de préférence entre 4 et 7.

11. Association selon l'une quelconque des revendications 1 à 10, caractérisée par le fait que les composants (A) et (B) se présentent sous forme de crèmes, de liquides plus ou moins épaissis, de dispersions vésiculaires de liquides amphiphiles ioniques ou non ioniques, de compositions aérosols ou d'huiles.

12. Association selon l'une quelconque des revendications 1 à 11, caractérisée par le fait que les composants (A) et (B) contiennent en plus des agents tensio-actifs, des agents hydratants, adoucissants, opacifiants, des vitamines, des parfums ou des agents conservateurs.

13. Dispositif à plusieurs compartiments ou "kit" ou "nécessaire" contenant des composants pouvant conférer à la peau une coloration similaire à la coloration résultant qu'un bronzage naturel, caractérisé par le fait qu'il comprend un premier compartiment contenant le composant (A) renfermant, dans un milieu approprié pour une application topique, la dihydroxyacétone, tel que défini dans la revendication 1, et un second compartiment contenant le composant (B) renfermant dans un milieu approprié pour une application topique, au moins un monohydroxyindole de formule (I) défini dans la revendication 1.

14. Dispositif à plusieurs compartiments ou "kit" ou "nécessaire" contenant des composants pour conférer à la peau une coloration similaire à celle de la coloration résultant d'un bronzage naturel, caractérisé par le fait qu'il est constitué par un dispositif à deux poches séparées réunies dans un étui souple, la première poche renfermant le composant (A) tel que défini dans la revendication 1, et la seconde poche renfermant le composant (B) tel que défini dans la revendication 1.

15. Procédé cosmétique de coloration de la peau afin de lui conférer une coloration similaire à celle d'un bronzage naturel, caractérisé par le fait que l'on applique sur la peau l'association telle que définie dans l'une quelconque des revendications 1 à 12.

## Patentansprüche

1. Mischung zur Hautfärbung, die der aus einer natürlichen Bräunung resultierenden Färbung ähnlich ist,
dadurch **gekennzeichnet**, daß
sie umfaßt:
einen Bestandteil (A), enthaltend in einem zur topischen Aufbringung geeigneten Medium Dihydroxyaceton, und
einen Bestandteil (B), enthaltend in einem für die Haut geeigneten Medium mindestens ein Monohydroxyindol gemäß der Formel: 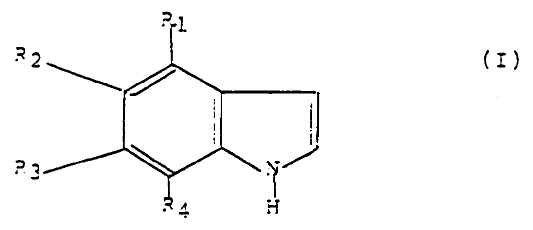 worin einer der vier Substituenten R₁, R₂, R₃ und R₄ OH bezeichnet, wobei die anderen drei Wasserstoff bezeichnen, sowie Additionssalze dieser Monohydroxyindole;
wobei die Bestandteile (A) und (B) Teile ein und derselben Zusammensetzung darstellen oder dazu bestimmt sind, entweder kurz vor der Anwendung vermischt oder nacheinander oder nach einem Ablauf von Zeit aufgebracht zu werden.

2. Mischung gemäß Anspruch 1,
dadurch **gekennzeichnet**, daß
die Bestandteile (A) und (B) eine einzige Zusammensetzung darstellen, enthaltend 0,1 bis 10 Gew.% und vorzugsweise 1 bis 6 Gew.% Dihydroxyaceton und 0,1 bis 10 Gew.%, vorzugsweise 0,1 bis 5 Gew.%,Monohydroxyindol der Formel (I), wobei diese Prozentangaben bezogen auf das Gesamtgewicht der Zusammensetzung ausgedrückt sind.

3. Mischung gemäß Anspruch 1,
dadurch **gekennzeichnet**, daß
die Bestandteile (A) und (B) getrennt voneinander vorliegen, wobei der Bestandteil (A) das Dihydroxyaceton in Mengenverhältnissen von 0,1 bis 10 Gew.% und insbesondere von 1 bis 6 Gew.%, bezogen auf Gesamtgewicht des Bestandteils (A), und der Bestandteil (B) das Monohydroxyindol der Formel (I) in Mengenverhältnissen von 0,1 bis 10 Gew.% und vorzugsweise von 0,1 bis 5 Gew.%, bezogen auf Gesamtgewicht der Zusammesnetzung (B), enthalten.

4. Mischung gemäß jedem Anspruch 1 bis 3,
dadurch **gekennzeichnet**, daß
das Gewichtsverhältnis zwischen dem Monohydroxyindol der Formel (I) und dem Dihydroxyaceton über 0,01 liegt.

5. Mischung gemäß jedem Anspruch 1 bis 4,
dadurch **gekennzeichnet**, daß
das zur topischen Aufbringung geeignete Medium ein Medium ist, enthaltend Wasser, eine Mischung aus Wasser und mindestens einem oder mehreren organischen Lösungsmittel(n) eine Mischung aus organischen Lösungsmitteln oder Fettsäurekörper, wobei die letzteren und die organischen Lösungsmittel kosmetisch zulässig sind.

6. Mischung gemäß jedem Anspruch 1 bis 5,
dadurch **gekennzeichnet**, daß
die Lösungsmittel ausgewählt sind aus C₁₋₄-Niedrigalkoholen, gesättigten Monoalkoholen mit langer Kette mit 10 bis 18 Kohlenstoffatomen, Polyolen mit 2 bis 8 Kohlenstoffatomen, Alkylenglycolen, Ethern von Glycolen, Essigsäureestern von C₁₋₃-Monoalkylethern von Ethylenglycol, Estern von gesättigten Fettsäuren mit 12 bis 14 Kohlenstoffatomen und gesättigten Alkohlen von 1 bis 4 Kohlenstoffatomen.

7. Mischung gemäß jedem Anspruch 1 bis 6,
dadurch **gekennzeichnet**, daß
die Lösungsmittel ausgewählt sind aus Ethyl-, Cetylalkohol, Propylenglycol, Ethylenglycolmonoethylether und Ethylenglycolmonobutylether.

8. Mischung gemäß jedem Anspruch 1 bis 7,
dadurch **gekennzeichnet**, daß
die Fettkörper ausgewählt sind aus mineralischen, pflanzlichen oder tierischen Ölen, Fettsäuren, Fettalkohlen, Fettsäureestern, Triglyceriden von Fettsäuren.

9. Mischung gemäß jedem Anspruch 1 bis 8,
dadurch **gekennzeichnet**, daß
die zur topischen Aufbringung geeigneten Medien verdickt sind.

10. Mischung gemäß jedem Anspruch 1 bis 9,
dadurch **gekennzeichnet**, daß
der pH des Bestandteils (A) 3 bis 10 und vorzugsweise 3 bis 7 und der pH des Bestandteils (B) 3 bis 10 und vorzugsweise 4 bis 7 betragen.

11. Mischung gemäß jedem Anspruch 1 bis 10,
dadurch **gekennzeichnet**, daß
die Bestandteile (A) und (B) in Form von Cremes, mehr oder weniger verdickten Flüssigkeiten, bläschenartigen Dispersionen von ionischen oder nicht-ionischen amphiphilen Lipiden, Aerosol-Zusammensetzungen oder in Form von Ölen vorliegen.

12. Mischung gemäß jedem Anspruch 1 bis 11,
dadurch **gekennzeichnet**, daß
die Bestandteile (A) und (B) zusätzlich oberflächenaktive Mittel, Hydratisiermittel, Weichmittel, opak-machende Mittel, Vitamine, Parfüms oder Konservierungsmittel enthalten.

13. Vorrichtung aus mehreren Teilen oder "Kit" oder "Necessaire", enthaltend Bestandteile, die der Haut eine Färbung verleihen können, die der aus einer natürlichen Bräunung resultierenden Färbung ähnlich ist,
dadurch **gekennzeichnet**, daß
sie einen ersten Teil, enthaltend den Bestandteil (A), umfasend, in einem für eine topische Aufbringung geeigneten Medium, das in Anspruch 1 definierte Dihydroxyaceton, und einen zweiten Teil aufweisen, enthaltend den Bestandteil (B), umfassend, in einem für eine topische Aufbringung geeigneten Medium, mindestens ein in Anspruch 1 definiertes Monohydroxyindol der Formel (I).

14. Vorrichtung aus mehreren Teilen oder "Kit" oder "Necessaire", enthaltend Bestandteile, um der Haut eine Färbung zu verleihen, die der aus einer natürlichen Bräunung resultierenden Färbung ähnlich ist,
dadurch **gekennzeichnet**, daß
sie aus einer Vorrichtung aus zwei getrennten Fächern, die in einem biegsamen Etui zusammengefaßt sind, zusammengesetzt sind, wobei das erste Fach den in Anspruch 1 definierten Bestandteil (A) und das zweite Fach den in Anspruch 1 definierten Bestandteil (B) umfassen.

15. Kosmetisches Verfahren für die Färbung der Haut, um ihr eine Färbung zu verleihen, die derjenigen aus einer natürlichen Bräunung ähnlich ist,
dadurch **gekennzeichnet**, daß
man auf die Haut, die gemäß jedem der Ansprüche 1 bis 12 definierte Mischung aufträgt.

## Claims

1. Combination intended for imparting to the skin a coloration similar to the coloration resulting from a natural tanning, characterised in that it comprises:
a) a component (A) containing dihydroxyacetone in a medium suitable for topical application; and
b) a component (B) containing, in a medium suitable for topical application, at least one monohydroxyindole corresponding to the formula: 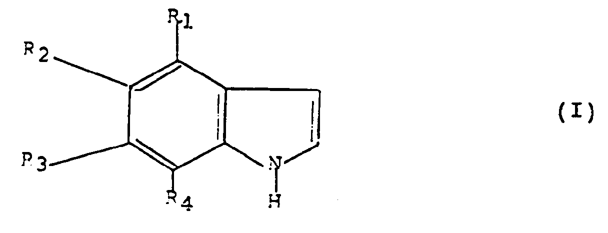 in which one of the four substituents R₁, R₂, R₃ and R₄ denotes OH, the other three denoting hydrogen,
as well as the addition salts of these monohydroxyindoles;
the components (A) and (B) forming part of one and the same composition, or being designed either to be mixed immediately before use or to be applied successively or with an intervening lapse of time.

2. Combination according to Claim 1, characterised in that the components (A) and (B) constitute a single composition containing 0.1 to 10 % by weight, and preferably 1 to 6 % by weight, of dihydroxyacetone, and 0.1 to 10 % by weight, and preferably 0.1 to 5 % by weight, of monohydroxyindole of formula (I), these percentages being expressed relative to the total weight of the composition.

3. Combination according to Claim 1, characterised in that the components (A) and (B) are separate, the component (A) containing dihydroxyacetone in proportions of between 0.1 and 10 % by weight, and especially between 1 and 6 % by weight, relative to the total weight of the component (A), and the component (B) containing the monohydroxyindole of formula (I) in proportions of between 0.1 and 10 % by weight, and preferably between 0.1 and 5 % by weight, relative to the total weight of the composition (B).

4. Combination according to any one of Claims 1 to 3, characterised in that the weight ratio of the monohydroxyindole of formula (I) to dihydroxyacetone is greater than 0.01.

5. Combination according to any one of Claims 1 to 4, characterised in that the medium suitable for topical application is a medium comprising water, a mixture of water and one or more organic solvent(s), a mixture of organic solvent or fats, the latter and the organic solvents being cosmetically acceptable.

6. Combination according to any one of Claims 1 to 5, characterised in that the solvents are selected from C₁-C₄ lower alcohols, saturated long-chain monohydric alcohols having 10 to 18 carbon atoms, polyols having 2 to 8 carbon atoms, alkylene glycols, glycol ethers, acetic esters of ethylene glycol mono(C₁-C₃ alkyl) ethers, and esters of saturated fatty acids having 14 to 16 carbon atoms and saturated alcohols having 1 to 4 carbon atoms.

7. Combination according to any one of Claims 1 to 6, characterised in that the solvents are selected from ethyl alcohol, cetyl alcohol, propylene glycol, ethylene glycol monoethyl ether and ethylene glycol monobutyl ether.

8. Combination according to any one of Claims 1 to 7, characterised in that the fats are selected from mineral, vegetable or animal oils, fatty acids, fatty alcohols, fatty acid esters and fatty acid triglycerides.

9. Combination according to any one of Claims 1 to 8, characterised in that the media suitable for topical application are thickened.

10. Combination according to any one of Claims 1 to 9, characterised in that the pH of the component (A) is between 3 and 10, and preferably between 3 and 7, and that the pH of the component (B) is between 3 and 10, and preferably between 4 and 7.

11. Combination according to any one of Claims 1 to 10, characterised in that the components (A) and (B) take the form of creams, more or less thickened liquids, vesicular dispersions of ionic or nonionic amphiphilic lipids, aerosol compositions or oils.

12. Combination according to any one of Claims 1 to 11, characterised in that the components (A) and (B) contain, in addition, surfactants, hydrating agents, demulcents, opacifiers, vitamins, fragrances or preservatives.

13. Multi-compartment device or "kit" or "oufit" containing components capable of imparting to the skin a coloration similar to the coloration resulting from a natural tanning, characterised in that it comprises a first compartment containing the component (A) containing dihydroxyacetone as defined in Claim 1, in a medium suitable for topical application, and a second compartment containing the component (B) containing, in a medium suitable for topical application , at least one monohydroxyindole of formula (I) defined in Claim 1.

14. Multi-compartment device or "kit or "outfit" containing components for imparting to the skin a coloration similar to the coloration resulting from a natural tanning , characterised in that it consists of a device having two separate bags combined in a flexible case, the first bag containing the component (A) as defined in Claim 1 and the second bag containing the component (B) as defined in Claim 1.

15. Cosmetic process for coloration of the skin in order to impart thereto a coloration similar to that of a natural tan, characterised in that the combination as defined in any one of Claims 1 to 12 is applied to the skin.
